# EUROPEAN PATENT APPLICATION

(11) **EP 3 333 154 A1**
(43) Date of publication of application: **13.06.2018**
(21) Application number: 16202760.1
(22) Date of filing: 07.12.2016
(51) Int. Cl.: C07D 209/14, A61K 31/4045, A61P 25/28

(54) **CRYSTALLINE FORM OF A SELECTIVE 5-HT6 RECEPTOR ANTAGONIST**

(71) Applicant: SANDOZ AG, 4056 Basel (CH)
(72) Inventor: LENGAUER, Hannes, 6250 Kundl (AT); PICHLER, Arthur, 6250 Kundl (AT); MARGREITER, Renate, 6250 Kundl (AT)
(74) Representative: Prüfer & Partner mbB Patentanwälte · Rechtsanwälte

(57) **Abstract**

The invention relates to a crystalline form of idalopirdine hydrochloride (form V) and to a process for its preparation. Furthermore, the invention relates to a pharmaceutical composition comprising the crystalline form V of idalopirdine hydrochloride of the present invention and to the use of said pharmaceutical composition as a medicament, in particular for the treatment of symptoms associated with Alzheimer's disease.

## Description

### FIELD OF THE INVENTION

The invention relates to a crystalline form of idalopirdine hydrochloride (form V) and to a process for its preparation. Furthermore, the invention relates to a pharmaceutical composition comprising the crystalline form V of idalopirdine hydrochloride of the present invention and to the use of said pharmaceutical composition as a medicament, in particular for the treatment of symptoms associated with Alzheimer's disease.

### BACKGROUND OF THE INVENTION

Idalopirdine is a selective antagonist of the 5-hydroxytryptamine receptor 5-HT₆, a receptor that is primarily expressed in areas of the brain that are involved in cognition and is thought to be involved in learning and memory (see Alzheimers Res. Ther. 5, 15; 2013). The compound is currently investigated in phase III clinical trials as symptomatic adjunctive therapy to cholinesterase inhibitors for the treatment of patients with mild to moderate Alzheimer's disease. Idalopirdine may chemically be designated *N*-[2-(6-fluoro-1*H*-indol-3-yl)ethyl]-3-(2,2,3,3-tetrafluoropropoxy)benzylamine and can be represented by the chemical structure according to Formula (I):

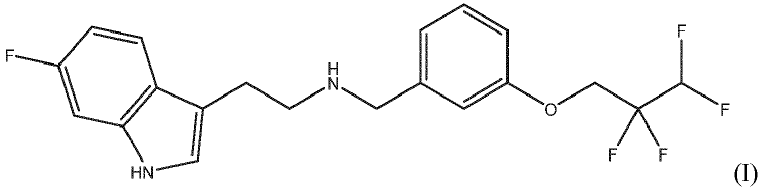

WO 02/078693 A2 discloses a general process for the preparation of compounds like idalopirdine in Example 370. By using the method of Example 370 idalopirdine was prepared and isolated as a hydrochloride salt in Example 376.

WO 2011/076212 A2 relates to a process for the preparation of idalopirdine. In example 4 of WO 2011/076212 A2 idalopirdine hydrochloride is described as being obtained as a white solid in >99.5% purity.

WO 2016/001398 A1 mentions on page 2, lines 9-10 that by using the methods of US 7,157,488 (which is a national stage entry of PCT application WO 02/078693 A2) and WO 2011/076212 A2, idalopirdine hydrochloride is obtained as a solid. On page 14, lines 21-23 the application states that the solids are polymorphic form I and form II of idalopridine hydrochloride. The application WO 2016/001398 A1 itself concerns a polymorphic form of idalopirdine hydrochloride denominated "form III", which it describes to be the thermodynamically most stable form. A so called "form IV" was only obtained as mixture with said form III.

WO 2016/078587 A1 discloses a crystalline form of idalopirdine hydrochloride designated form A. The PXRD of said form A, which is provided in figure 1 of WO 2016/078587 corresponds well to the diffractogram of form II provided in figure 3 of WO 2016/001398 A1.

On page 6 WO 2016/001398 A1 explains that it is difficult to obtain polymorphic forms of idalopirdine hydrochloride other than forms I and II. According to WO 2016/001398 A1 page 6, lines 4 to 6, crystallization experiments have been performed in the search for other polymorphic modifications of idalopirdine, and all experiments resulted in either polymorphic form I or II, depending on the temperature. The fact that the crystalline form disclosed in WO 2016/078587 A1 also corresponds to form II may support this conclusion. Form III was prepared by long-term slurrying of idalopirdine form I in defined solvent systems or by seeding.

Since different polymorphic forms of a drug substance can possess different physical properties, the provision of further polymorphic forms of idalopirdine hydrochloride would increase the repertoire of materials available for a formulation scientist and would allow for the development of more diverse and customized pharmaceutical compositions. In particular, the provision of a polymorphic form having improved physicochemical properties such as high solubility and dissolution rate in physiologically relevant media, good chemical and physical stability, low hygroscopicity and good powder properties allowing for convenient pharmaceutical processing would be desirable. Beside the appropriate physicochemical properties of a solid form, its reliable and economic manufacturability is also an important criterion for selecting a particular polymorphic form.

### SUMMARY OF THE INVENTION

The present invention provides a crystalline form of idalopirdine hydrochloride, herein also designated "form V", which is characterized by having a powder X-ray diffractogram comprising reflections at 2-theta angles of (2.30 ± 0.1)°, (4.63 ± 0.1)°, (18.78 ± 0.1)°, (19.69 ± 0.1)° and (25.24 ± 0.1)°, when measured at RT with Cu-Kalpha_{1,2} radiation having a wavelength of 0.15419 nm. The invention further relates to a process for the preparation of crystalline form V of idalopirdine hydrochloride of the present invention. Moreover, the present invention relates to a pharmaceutical composition comprising the crystalline form V of idalopirdine hydrochloride of the present invention, preferably in an effective and/or predetermined amount, and one or more pharmaceutically acceptable excipients. The present invention also relates to the use of said pharmaceutical composition as a medicament, in particular for the treatment of symptoms associated with Alzheimer's disease.

### Abbreviations

- PXRD: powder X-ray diffractogram
- FTIR: Fourier transform infrared
- ATR: attenuated total reflection
- DSC: differential scanning calorimetry
- TGA: thermogravimetric analyses
- GMS: gravimetric moisture sorption
- RH: relative humidity
- RT: room temperature
- w-%: weight percent
- v-%: volume percent

### Definitions

The term "idalopirdine" as used herein refers to the compound with the chemical name *N*-[2-(6-fluoro-1*H*-indol-3-yl)ethyl]-3-(2,2,3,3-tetrafluoropropoxy)benzylamine, which is represented by the chemical structure as depicted in Formula I of the present invention.

The term "idalopirdine hydrochloride" refers to the the compound with the chemical name *N*-[2-(6-fluoro-1*H*-indol-3-yl)ethyl]-3-(2,2,3,3-tetrafluoropropoxy)benzylamine hydrochloride, which is represented by the chemical structure as depicted in Formula II of the present invention. In the context of the present invention the term "idalopirdine hydrochloride" means the monohydrochloride salt of idalopirdine, which is characterized by having a molar ratio of idalopirdine and hydrochloric acid of from 1.0: 0.7 to 1.0: 1.3, preferably from 1.0: 0.8 to 1.0: 1.2, more preferably from 1.0: 0.9 to 1.0: 1.1 and most preferably the molar ratio of idalopirdine and hydrochloric acid is 1.0: 1.0.

In the context of a polymorphic or crystalline form of idalopirdine hydrochloride, the term "form I" as used herein refers to the crystalline form of idalopirdine hydrochloride, which is implicitly disclosed in WO 02/078693 A2 and characterized by having essentially the same PXRD as displayed in Figure 1 of WO 2016/001398 A1, when measured at RT with Cu-Kalpha_{1,2} radiation having a wavelength of 0.15419 nm.

In the context of a polymorphic or crystalline form of idalopirdine hydrochloride, the term "form II" as used herein refers to the crystalline form of idalopirdine hydrochloride, which is implicitly disclosed in WO 02/078693 A2 and characterized by having essentially the same PXRD as displayed in Figure 3 of WO 2016/001398 A1, when measured at RT with Cu-Kalpha_{1,2} radiation having a wavelength of 0.15419 nm.

In the context of a polymorphic or crystalline form of idalopirdine hydrochloride, the term "form III" as used herein refers to the crystalline form of idalopirdine hydrochloride of WO 2016/001398 A1, which can be characterized by having a PXRD comprising reflections at 2-Theta angles of (4.63 ± 0.1)°, (6.94 ± 0.1)°, (13.89 ± 0.1)°, (17.26 ± 0.1)° and (19.97 ± 0.1)°, when measured at RT with Cu-Kalpha_{1,2} radiation having a wavelength of 0.15419 nm.

As used herein the term "room temperature" refers to a temperature in the range of from 20 to 30 °C.

As used herein, the term "measured at a temperature in the range of from 20 to 30 °C" refers to a measurement under standard conditions. Typically, standard conditions mean a temperature in the range of from 20 to 30 °C, i.e. at room temperature. Standard conditions are preferably a temperature of about 22 °C and a measurement at 25% to 60% RH, such as at about 50% RH.

As used herein, the term "reflection" with regards to powder X-ray diffraction means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 103 to 1020 atoms, whereas short-range order is over a few atoms only (see *"*Fundamentals of Powder Diffraction and Structural Characterization of Materials" " by Vitalij K. Pecharsky and Peter Y. Zavalij, Kluwer Academic Publishers, 2003, page 3).

The term "essentially the same" with reference to powder X-ray diffractometry means that variabilities in the positions and relative intensities of reflections are to be taken into account. For example, a typical precision of the 2-Theta values is in the range of ± 0.1 2-Theta. Thus, a diffraction peak that usually appears at 2.32° 2-Theta for example can appear between 2.22° and 2.42° 2-Theta on most X-ray diffractometers under standard conditions. Furthermore, one skilled in the art will appreciate that relative peak intensities will show inter-apparatus variability as well as variability due to degree of crystallinity, preferred orientation, sample preparation and other factors known to those skilled in the art and should be taken as qualitative measure only.

The term "essentially the same" with reference to FTIR spectroscopy means that variabilities in peak positions and relative intensities of the peaks are to be taken into account. For example, a typical precision of the wavenumber values is in the range of ± 2 cm⁻¹. Thus, a peak at 3426 cm⁻¹ for example can appear in the range of from 3424 to 3428 cm⁻¹ on most infrared spectrometers under standard conditions. Differences in relative intensities are typically smaller compared to X-ray diffraction. However, one skilled in the art will appreciate that small differences in peak intensities due to degree of crystallinity, sample preparation and other factors can also occur in infrared spectroscopy. Relative intensities should therefore be taken as qualitative measure only.

Crystalline form V of idalopirdine hydrochloride of the present invention may be referred to herein as being characterized by graphical data "as shown in" a figure. Such data include, for example, PXRDs and FTIR spectra. The person skilled in the art understands that factors such as variations in instrument type, response and variations in sample directionality, sample concentration and sample purity may lead to small variations for such data when presented in graphical form, for example variations relating to the exact peak positions and intensities.

A "predetermined amount" as used herein with regard to the crystalline form V of idalopirdine hydrochloride of the present invention refers to the initial amount of said crystalline form V used for the preparation of a pharmaceutical composition.

The term "effective amount" as used herein with regard to crystalline form V of idalopirdine hydrochloride of the present invention encompasses an amount of said crystalline form V in a unit dosage form, which causes the desired therapeutic and/or prophylactic effect when the unit dosage form is administered according to a suitable dosage regimen.

The term "non-hygroscopic" as used herein refers to material, such as a crystalline compound, which shows a water uptake of at most 2.0 w-%, preferably of at most 1.0 w-% and most preferably of at most 0.5 w-% in the sorption cycle when said material, such as the crystalline compound, is measured with gravimetric moisture sorption at a relative humidity in the range of from 0 to 95% and a temperature of (25.0 ± 0.1) °C, based on the weight of the compound. As used herein, the term "about" means within a statistically meaningful range of a value. Such a range can be within an order of magnitude, typically within 10%, more typically within 5%, even more typically within 1% and most typically within 0.1 % of the indicated value or range. Sometimes, such a range can lie within the experimental error, typical of standard methods used for the measurement and/or determination of a given value or range.

As used herein, the term "mother liquor" refers to the solution remaining after crystallization of a solid.

The term "antisolvent" as used herein refers to liquids which reduce the solubility of idalopirdine hydrochloride in a solvent.

### BRIEF DESCRIPTION OF THE DRAWINGS

**Figure 1****:** illustrates a representative PXRD of crystalline form V of idalopirdine hydrochloride according to the present invention. The x-axis shows the scattering angle in °2-theta, the y-axis shows the intensity of the scattered X-ray beam in counts of detected photons.
**Figure 2****:** illustrates a representative FTIR spectrum of crystalline form V of idalopirdine hydrochloride according to the present invention. The x-axis shows the wavenumbers in cm⁻¹, the y-axis shows the relative intensity in percent transmittance.
**Figure 3****:** illustrates a representative DSC curve of crystalline form V of idalopirdine hydrochloride according to the present invention. The x-axis shows the temperature in degree Celsius (°C), the y-axis shows the heat flow in Watt per gram (W/g) with endothermic peaks going up.
**Figure 4****:** illustrates a representative TGA curve of crystalline form V of idalopirdine hydrochloride according to the present invention. The x-axis shows the temperature in degree Celsius (°C), the y-axis shows the mass loss in weight-percent (w-%)

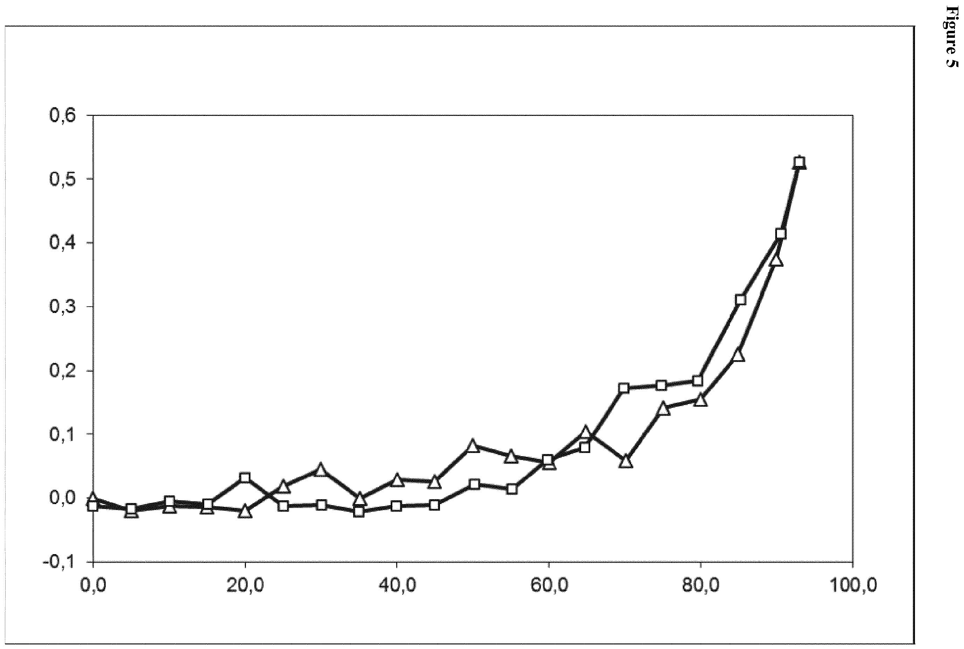

**Figure 5****:** illustrates representative gravimetric moisture sorption and desorption curves of crystalline form V of idalopirdine hydrochloride of the present invention in the range of from 0 to 95% relative humidity. The x-axis displays the relative humidity in percent (%) measured at a temperature of (25.0 ± 0.1) °C, the y-axis displays the equilibrium mass change in weight percent (w-%). The sorption cycle is marked by triangles, whereas the desorption cycle is marked by squares. The values are displayed as uncorrected values, whereat the weight at 0% relative humidity has been set to zero as starting weight.
**Figure 6****:** illustrates a comparison of the dissolution curves of idalopirdine hydrochloride form V of the present invention (upper curve marked as squares) and idalopirdine hydrochloride form III of WO 2016/001398 A1 (lower curve marked as triangles) in aqueous 0.1 N HCl solution at
a temperature of (25 ± 1) °C. The x-axis shows the time in minutes (min), the y-axis shows the concentration in milligram per milliliter (mg/mL).

### DETAILED DESCRIPTION OF THE INVENTION

Different aspects of the invention are described below in further detail by embodiments, without being limited thereto. Each aspect of the invention may be described by one embodiment or by combining two or more of the embodiments.

The present invention provides a polymorphic form of idalopirdine hydrochloride, herein also designated as "form V".

Idalopirdine hydrochloride may be represented by the chemical structure according to Formula II: 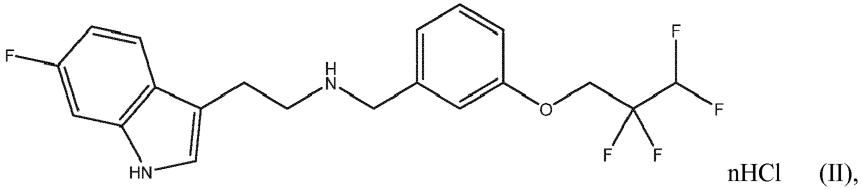 wherein n is in the range of from 0.7 to 1.3, preferably from 0.8 to 1.2, more preferably from 0.9 to 1.1 and most preferably n is 1.0. For example, n is selected from the group consisting of 0.7,0.8,0.9, 1.0, 1.1, 1.2 and 1.3.

Polymorphic form V of idalopirdine hydrochloride of the present invention is chemically and physically stable over a broad humidity and temperature range and upon light exposure and shows high solubility and dissolution in physiologically relevant media. In addition, it is non-hygroscopic and shows favorable powder properties such as, but not limited to, good flowability and compressibility, high bulk density and low dusting. Moreover, form V can be produced in a reliable and economic manner also on industrial scale. In view of its excellent physicochemical properties and due to the fact that form V can be manufactured in a reliable and robust manner, this form is particularly suited for pharmaceutical purposes, such as formulation into a tablet or capsule.

Hence, in a first aspect, the invention relates to a crystalline form of idalopirdine hydrochloride (form V) characterized by having a PXRD comprising reflections at 2-Theta angles of:
(2.30 ± 0.1)° and (19.69 ± 0.1)°; or
(2.30 ± 0.1)°, (18.78 ± 0.1)° and (19.69 ± 0.1)°; or
(2.30 ± 0.1)°, (18.78 ± 0.1)°, (19.69 ± 0.1)° and (25.24 ± 0.1)°; or
(2.30 ± 0.1)°, (4.63 ± 0.1)°, (18.78 ± 0.1)°, (19.69 ± 0.1)° and (25.24 ± 0.1)°; or
(2.30 ± 0.1)°, (4.63 ± 0.1)°, (18.78 ± 0.1)°, (19.69 ± 0.1)°, (21.62 ± 0.1)° and (25.24 ± 0.1)°; or
(2.30 ± 0.1)°, (4.63 ± 0.1)°, (13.95 ± 0.1)°, (18.78 ± 0.1)°, (19.69 ± 0.1)°, (21.62 ± 0.1)° and (25.24 ± 0.1)°; or
(2.30 ± 0.1)°, (4.63 ± 0.1)°, (13.95 ± 0.1)°, (17.95 ± 0.1)°, (18.78 ± 0.1)°, (19.69 ± 0.1)°, (21.62 ± 0.1)° and (25.24 ± 0.1)°; or
(2.30 ± 0.1)°, (4.63 ± 0.1)°, (13.95 ± 0.1)°, (17.95 ± 0.1)°, (18.78 ± 0.1)°, (19.69 ± 0.1)°, (21.62 ± 0.1)°, (23.07 ± 0.1)° and (25.24 ± 0.1)°; or
(2.30 ± 0.1)°, (4.63 ± 0.1)°, (13.95 ± 0.1)°, (17.20 ± 0.1)°, (17.95 ± 0.1)°, (18.78 ± 0.1)°, (19.69 ± 0.1)°, (21.62 ± 0.1)°, (23.07 ± 0.1)° and (25.24 ± 0.1)°;
when measured at RT with Cu-Kalpha_{1,2} radiation having a wavelength of 0.15419 nm.

Powder X-ray diffraction is a powerful means for differentiating crystalline form V of idalopirdine hydrochloride from other crystalline forms of the compound. For example, in contrast to the PXRD of form V, the PXRDs of form I and form III do not show reflections in the range of from (19.69 ± 0.1)° 2-Theta and the PXRD of form II is lacking reflections in the range of from (2.30 ± 0.1)°. On the other hand, form III for example shows reflections in the range of from (18.49 ± 0.1)°, (19.47 ± 0.1)°, (19.97 ± 0.1)°, (21.83 ± 0.1)°, (22.53 ± 0.1)°, (23.65 ± 0.1)° and (28.91 ± 0.1)° 2-Theta, whereas form V of the present invention does not display reflections in these ranges.

In another embodiment the present invention therefore relates to crystalline form V of idalopirdine hydrochloride, characterized by having a PXRD comprising reflections at the 2-Theta angles of:
(2.30 ± 0.1)° and (19.69 ± 0.1)°; or
(2.30 ± 0.1)°, (18.78 ± 0.1)° and (19.69 ± 0.1)°; or
(2.30 ± 0.1)°, (18.78 ± 0.1)°, (19.69 ± 0.1)° and (25.24 ± 0.1)°; or
(2.30 ± 0.1)°, (4.63 ± 0.1)°, (18.78 ± 0.1)°, (19.69 ± 0.1)° and (25.24 ± 0.1)°; or
(2.30 ± 0.1)°, (4.63 ± 0.1)°, (18.78 ± 0.1)°, (19.69 ± 0.1)°, (21.62 ± 0.1)° and (25.24 ± 0.1)°; or
(2.30 ± 0.1)°, (4.63 ± 0.1)°, (13.95 ± 0.1)°, (18.78 ± 0.1)°, (19.69 ± 0.1)°, (21.62 ± 0.1)° and (25.24 ± 0.1)°; or
(2.30 ± 0.1)°, (4.63 ± 0.1)°, (13.95 ± 0.1)°, (17.95 ± 0.1)°, (18.78 ± 0.1)°, (19.69 ± 0.1)°, (21.62 ± 0.1)° and (25.24 ± 0.1)°; or
(2.30 ± 0.1)°, (4.63 ± 0.1)°, (13.95 ± 0.1)°, (17.95 ± 0.1)°, (18.78 ± 0.1)°, (19.69 ± 0.1)°, (21.62 ± 0.1)°, (23.07 ± 0.1)° and (25.24 ± 0.1)°; or
(2.30 ± 0.1)°, (4.63 ± 0.1)°, (13.95 ± 0.1)°, (17.20 ± 0.1)°, (17.95 ± 0.1)°, (18.78 ± 0.1)°, (19.69 ± 0.1)°, (21.62 ± 0.1)°, (23.07 ± 0.1)° and (25.24 ± 0.1)°;
when measured at RT with Cu-Kalpha_{1,2} radiation having a wavelength of 0.15419 nm, but not comprising a reflection at (a) 2-Theta angle(s) of (18.49 ± 0.1)°, (19.47 ± 0.1)°, (19.97 ± 0.1)°, (21.83 ± 0.1)°, (22.53 ± 0.1)°, (23.65 ± 0.1)° and/or (28.91 ± 0.1)°, such as not comprising a reflection at a 2-Theta angle of (19.97 ± 0.1)°.

For example the PXRD of form V of the present invention may be characterized by not comprising reflections at two positions selected from the group consisting of 2-Theta angles of (18.49 ± 0.1)°, (19.47 ± 0.1)°, (19.97 ± 0.1)°, (21.83 ± 0.1)°, (22.53 ± 0.1)°, (23.65 ± 0.1)° and (28.91 ± 0.1)°. More specifically the PXRD of form V of the present invention may be characterized by not comprising reflections at three positions selected from the group consisting of 2-Theta angles of (18.49 ± 0.1)°, (19.47 ± 0.1)°, (19.97 ± 0.1)°, (21.83 ± 0.1)°, (22.53 ± 0.1)°, (23.65 ± 0.1)° and (28.91 ± 0.1)°. Preferably the PXRD of form V of the present invention may be characterized by not comprising reflections at 2-Theta angles of (18.49 ± 0.1)°, (19.47 ± 0.1)°, (19.97 ± 0.1)°, (21.83 ± 0.1)°, (22.53 ± 0.1)°, (23.65 ± 0.1)° and (28.91 ± 0.1)°.

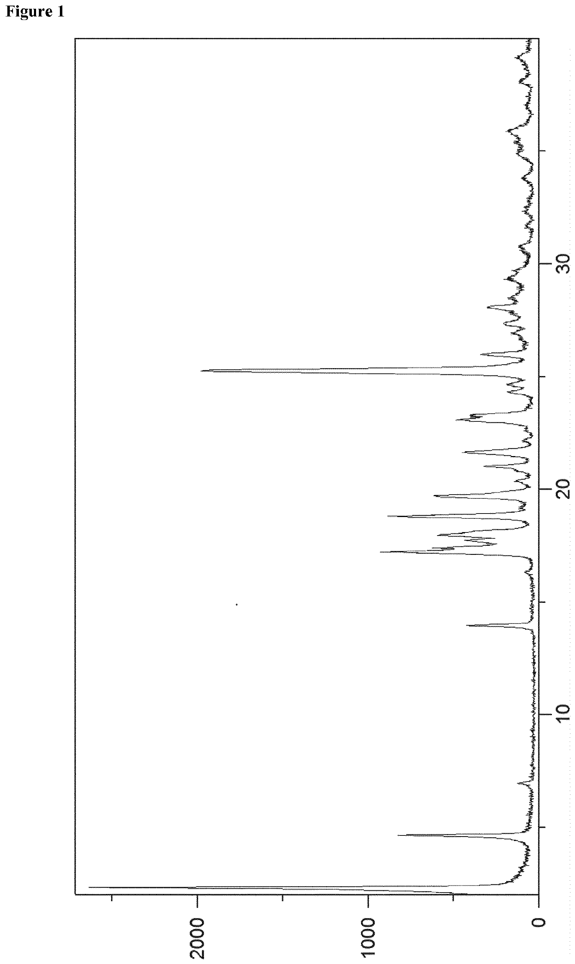

In still another embodiment, the present invention relates to a crystalline form of idalopirdine hydrochloride (form V) characterized by having a PXRD essentially the same as shown in Figure 1 of the present invention, when measured at RT with Cu-Kalpha_{1,2} radiation having a wavelength of 0.15419 nm.

In yet another embodiment, the present invention relates to a crystalline form of idalopirdine hydrochloride (form V) characterized by having an FTIR spectrum comprising peaks at wavenumbers of:
(3426 ± 2) cm⁻¹, (1586 ± 2) cm⁻¹, (1245 ± 2) cm⁻¹, (1045 ± 2) cm⁻¹ and (733 ± 2) cm⁻¹; or
(3426 ± 2) cm⁻¹, (1586 ± 2) cm⁻¹, (1245 ± 2) cm⁻¹, (1045 ± 2) cm⁻¹, (854 ± 2) cm⁻¹ and (733 ± 2) cm⁻¹; or
(3426 ± 2) cm⁻¹, (1586 ± 2) cm⁻¹, (1452 ± 2) cm⁻¹, (1245 ± 2) cm⁻¹, (1045 ± 2) cm⁻¹, (854 ± 2) cm⁻¹ and (733 ± 2) cm⁻¹; or
(3426 ± 2) cm⁻¹, (1586 ± 2) cm⁻¹, (1452 ± 2) cm⁻¹, (1245 ± 2) cm⁻¹, (1091 ± 2) cm⁻¹, (1045 ± 2) cm⁻¹, (854 ± 2) cm⁻¹ and (733 ± 2) cm⁻¹; or
(3426 ± 2) cm⁻¹, (1586 ± 2) cm⁻¹, (1452 ± 2) cm⁻¹, (1245 ± 2) cm⁻¹, (1091 ± 2) cm⁻¹, (1045 ± 2) cm⁻¹, (854 ± 2) cm⁻¹, (790± 2) and (733 ± 2) cm⁻¹; or
(3426 ± 2) cm⁻¹, (2952 ± 2) cm⁻¹, (1586 ± 2) cm⁻¹, (1452 ± 2) cm⁻¹, (1245 ± 2) cm⁻¹, (1091 ± 2) cm⁻¹, (1045 ± 2) cm⁻¹, (854 ± 2) cm⁻¹, (790± 2) and (733 ± 2) cm⁻¹;
when measured at RT with a diamond ATR cell.

FTIR spectroscopy is also a powerful means for differentiating crystalline form V of idalopirdine hydrochloride from forms II and III. The spectrum of form II is lacking peaks in the range of from (3426 ± 2) cm⁻¹, (1586 ± 2) cm⁻¹, (1245 ± 2) cm⁻¹ and (1045 ± 2) cm⁻¹ and the spectrum of form III shows no peaks in the range of from (1045 ± 2) cm⁻¹, (854 ± 2) cm⁻¹ and (733 ± 2) cm⁻¹, whereas form V displays peaks at these positions.

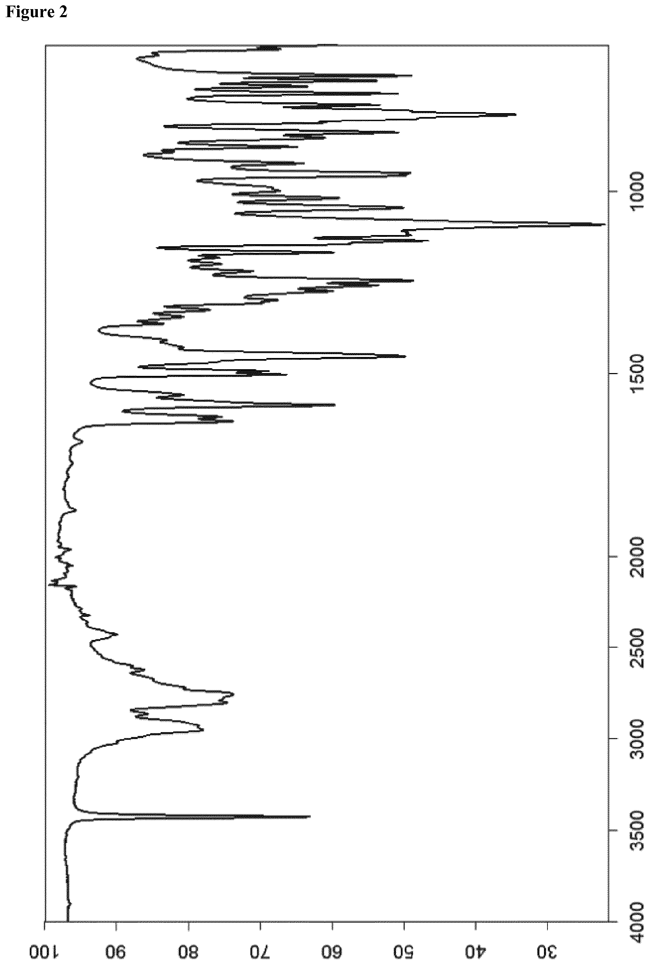

In still another embodiment, the present invention relates to a crystalline form of idalopirdine hydrochloride (form V) characterized by having an FTIR spectrum which is essentially the same as displayed in Figure 2 of the present invention, when measured at RT with a diamond ATR cell.

In order to differentiate form V of idalopirdine hydrochloride from other crystalline forms of the compound, characteristic PXRD reflections and FTIR peaks may be combined.

Hence, in a preferred embodiment, the invention relates to a crystalline form of idalopirdine hydrochloride (form V) characterized by having:
(i) a PXRD comprising reflections at 2-Theta angles of (2.30 ± 0.1)° and (19.69 ± 0.1)°, when measured at RT with Cu-Kalpha_{1,2} radiation having a wavelength of 0.15419 nm; and
(ii) an FTIR spectrum comprising peaks at wavenumbers of (3426 ± 2) cm⁻¹ and (1045 ± 2) cm⁻¹, when measured at RT with a diamond ATR cell.

In a further preferred embodiment, the invention relates to a crystalline form of idalopirdine hydrochloride (form V) characterized by having:
(i) a PXRD comprising reflections at 2-Theta angles of (2.30 ± 0.1)°, (18.78 ± 0.1)° and (19.69 ± 0.1)°, when measured at RT with Cu-Kalpha_{1,2} radiation having a wavelength of 0.15419 nm; and
(ii) an FTIR spectrum comprising peaks at wavenumbers of (3426 ± 2) cm⁻¹, (1045 ± 2) cm⁻¹ and (854 ± 2) cm⁻¹, when measured at RT with a diamond ATR cell.

In another embodiment, the invention relates to a crystalline form of idalopirdine hydrochloride characterized by having a DSC curve showing a first endothermic peak with an onset temperature in the range of from 130 to 140 °C, preferably from 130 to 135 °C, such as at about 132 °C, and a second endothermic peak with an onset temperature in the range of from 165 °C to 175 °C, preferably from 167 to 173 °C, such as at about 170 °C, when measured at a heating rate of 10 K/min.

In still another embodiment, the invention relates to a crystalline form of idalopirdine hydrochloride (form V) characterized by having a TGA curve showing a mass loss of at most 1.0 w-%, preferably of at most 0.8 w-%, even more preferably of at most 0.6 w-%, based on the weight of the crystalline form when measured from RT to 160 °C at a heating rate of 10 K/min.

In yet another embodiment, the present invention relates to a crystalline form of idalopirdine hydrochloride (form V) characterized by showing a mass change of at most 2.0 w-%, preferably of at most 1.0 w-% and most preferably of at most 0.5 w-%, based on the weight of the crystalline form, when measured with GMS at a RH in the range of from 0 to 95% and a temperature of (25.0 ± 0.1) °C.

In another aspect the invention relates to a composition comprising at least 90 w-%, preferably at least 95 w-%, such as at least 99 w-%, of the crystalline form of idalopirdine hydrochloride of the present invention (form V), based on the total weight of the composition.

It is preferred that the composition does not comprise idalopirdine hydrochloride form I, and/or idalopirdine hydrochloride form II, and/or idalopirdine hydrochloride form III. More preferably the composition does not comprise any of idalopirdine hydrochloride form I, form II and form III.

Idalopirdine hydrochloride form V of the present invention exhibits advantageous physicochemical properties, which render this form especially suitable for pharmaceutical purposes. For example, form V is chemically and physically highly stable. Even when subjected to accelerated stress conditions for a prolonged period (e.g. for one month), form V shows hardly any chemical degradation and also the PXRD remains unchanged, proving that no form transformation had occurred. The results of the stress test are summarized in Example 3 herein.

Furthermore, form V of the present invention shows very little interaction with water vapor, when subjected to a relative humidity in the range of from 0 to 95% at (25.0 ± 1.0) °C. As can be seen from Figure 5 herein, a mass increase of only 0.5 w-% in the sorption cycle from 0 to 95% shows that form V is non-hygroscopic. In addition, the lack of a significant hysteresis between the sorption and desorption curve indicates that no structural changes occurred during the experiment. This was confirmed by PXRD.

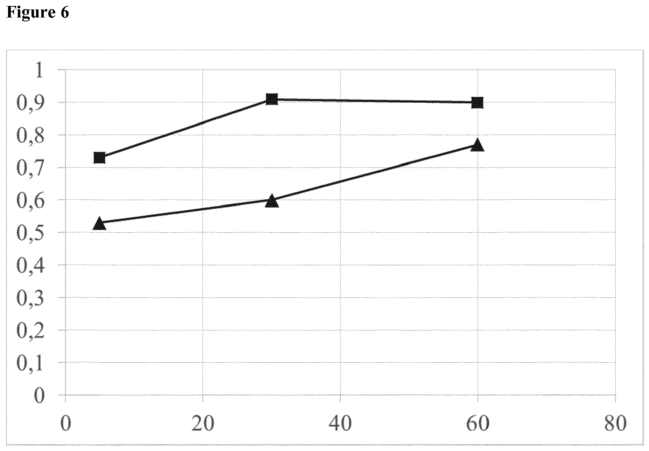

Furthermore, compared to form III of WO 2016/001398 A1, form V of the present invention shows an improved solubility and dissolution rate in aqueous 0.1 N HCl solution, a medium simulating the pH environment of the stomach (see Comparative Example 1 and Figure 6 of the present invention). It is well known to the person skilled in the art that solubility, dissolution rate and gastrointestinal permeability are fundamental parameters that control rate and extent of drug absorption and its bioavailability. The aqueous solubility of a drug is a crucial property that plays an important role in the absorption of the drug after oral administration. Since form V of idalopirdine hydrochloride shows up to 50% higher concentration in 0.1 N HCl this form is therefore expected to achieve higher blood levels after oral administration.

In still a further aspect, the invention relates to a process for the preparation of the crystalline form of idalopirdine hydrochloride as defined above (form V) comprising the steps of:
(a) providing a suspension comprising idalopirdine hydrochloride, 1,4-dioxane and one or more antisolvent(s), wherein 1,4-dioxane is present in an amount in the range of from 51 to 65 v-%, based on the total volume of the solvent/antisolvent mixture;
(b) heating the mixture provided in step (a) to a temperature of at least 60 °C to obtain a solution;
(c) cooling the solution provided in step (b) to a temperature in the range of from 5 to 30 °C, preferably from 0 to 10 °C to initiate crystallization;
(d) optionally adding idalopirdine hydrochloride form V seed crystals to the mixture provided in step (c);
(e) optionally separating at least a part of the crystals obtained in step (c) or (d) from their mother liquor;
(f) optionally drying the crystals obtained in any one of steps (c) to (e).

Idalopirdine hydrochloride, which is used as starting material in step (a) of the above described process can be prepared according to the procedure disclosed in Example 4 of WO 2011/076212 A2.

In a first step, a suspension comprising idalopirdine hydrochloride, 1,4-dioxane and one or more antisolvent(s) is provided. The amount of 1,4-dioxane is preferably in the range of from 51 to 65 v-%, more preferably from 52 to 55 v-%, based on the total volume of the solvent/antisolvent mixture. In a preferred embodiment, 1,4-dioxane is the only solvent present. Preferably, the one or more antisolvent(s) is selected from the group consisting of *n*-heptane, *n*-octane, cyclohexane, diisopropyl ether and *tert*-butylmethyl ether. Most preferably, the antisolvent is n-heptane. In a particular preferred embodiment, *n*-heptane is the only antisolvent present.

The suspension provided in step (a) is heated to a temperature of at least 60 °C, preferably of at least 80 °C, and most preferably the mixture is heated to reflux temperature in order to fully dissolve the starting material. The idalopirdine hydrochloride concentration of the obtained solution preferably is in the range of from about 30 to 60 g/L, more preferably from about 30 to 50 g/L and most preferably from about 30 to 40 g/L. The solution may optionally be filtered in order to remove any undissolved particles.

Crystallization of idalopirdine hydrochloride form V crystals can be initiated by cooling the solution obtained in step (b) to a temperature in the range of from about 5 to 30 °C, preferably from about 0 to 10 °C. The solution is preferably cooled at a cooling rate in the range of from about -10 to -1 K/min, preferably from about -10 to -5 K/min.

Optionally, idalopirdine hydrochloride seed crystals are added to promote crystallization and to control crystal growth. Seed crystals can be prepared according to Example 2 disclosed herein. The amount of seed crystals employed may range from about 0.1 w-% to 20.0 w-%, preferably from about 0.1 w-% to 10.0 w-% and most preferably from about 0.1 w-% to 1.0 w-%, based on the weight of the idalopiridine hydrochloride starting material. Once seed crystals have been added, the mixture may be further kept at the temperature of about 5 to 30 °C, preferably of about 0 to 10 °C with or without agitation, e.g. with or without stirring.

The obtained idalopirdine hydrochloride crystals may optionally be separated from their mother liquor by any conventional method such as filtration or centrifugation, most preferably by filtration. Optionally, the isolated crystals may be washed with one or more antisolvent(s). Preferably, the one or more antisolvent(s) which is/are used for the washing step is/are the same like the one or more antisolvent(s) applied in step (a) of the above described procedure, most preferably the antisolvent in both steps is *n*-heptane.

Finally, the idalopirdine hydrochloride form V crystals may optionally be dried at a temperature of about 80 °C or less, preferably of about 60 °C or less, more preferably of about 40 °C or less, and most preferably the crystals are dried at RT. Drying may be performed for a period in the range of from about 1 to 72 hours, preferably from about 2 to 48 hours, more preferably from about 4 to 24 hours and most preferably from about 6 to 18 hours. Drying may be performed at ambient pressure and/or under vacuum preferably at about 100 mbar or less, more preferably at about 50 mbar or less and most preferably at about 30 mbar or less, for example at about 20 mbar or less.

The process for form V production as described above is reliable and economic. In contrast, the processes for form III production as disclosed in WO 2016/001398 A2 suffer from certain drawbacks with regard to robustness and economics. For example, starting from form I, the solvent-mediated transformations in Example 2 of WO 2016/001398 A2 require that the suspensions containing form I are slurried for prolonged periods of time (see page 24, lines 6 to 7) e.g. from one week up to one month regardless of the temperature. Also the seeding processes described in Example 3 of the same application seem to require prolonged stirring such as the indicated 3 days. In addition, the application mentions in the paragraph bridging pages 6 and 7 that at the temperature, where polymorphic form III is the thermodynamically stable polymorphic form, it is easier to produce polymorphic forms I and II since they have a kinetic advantage over polymorphic form III, making this form more difficult to produce.

Manufacturability is a factor for the selection of a polymorphic form of a drug substance. Also in this regard form V of the present invention is preferred compared to form III of WO 2016/001398 A2, since form V can be produced in a more reliable and economic manner.

In another aspect, the present invention relates to the use of crystalline form V of idalopirdine hydrochloride as described herein for the preparation of a pharmaceutical composition.

In a further aspect, the present invention relates to a pharmaceutical composition comprising crystalline form V of idalopirdine hydrochloride as defined herein, preferably in an effective and/or predetermined amount, and one or more pharmaceutically acceptable excipient(s).

In a preferred embodiment, the predetermined and/or effective amount of crystalline form V of idalopirdine hydrochloride of the pharmaceutical composition as defined above is selected from the group consisting of 5 mg, 10 mg, 15 mg, 20 mg, 25 mg, 30 mg, 35 mg, 40 mg, 45 mg, 50 mg, 55 mg and 60 mg, calculated as idalopirdine. Most preferably, the predetermined and/or effective amount of crystalline form V of idalopirdine hydrochloride of the pharmaceutical composition as defined above is 30 mg or 60 mg, calculated as idalopirdine.

In a further preferred embodiment, the one or more pharmaceutically acceptable excipient(s) of the pharmaceutical composition as defined above is/are one or more diluent(s). Generally, diluents are used to top up the volume for an appropriate oral deliverable dose, when low concentrations of the active pharmaceutical ingredients (about 25 w-% or lower, based on the total amount of the pharmaceutical composition) are present. In powder formulations diluents can enhance the powder flow, facilitating, for example, a uniform filling of capsules.

Preferably, the one or more diluent(s) is/are selected from the group consisting of carbohydrates such as sugars, sugar alcohols, starches and celluloses. In another embodiment, the sugar may be selected from the group consisting of lactose, e.g. lactose monohydrate, anhydrous lactose or spray dried lactose, sucrose, dextrose, fructose, glucose, maltose and maltodextrin. In still another embodiment, the sugar alcohol may be selected from the group consisting of mannitol, sorbitol, xylitol and inositol. In a further embodiment the starches may be selected from corn starch and potato starch, whereas the starches are preferably pre-gelatinized or hydrolyzed. In yet another embodiment the celluloses may be selected from the group consisting of powdered cellulose, microcrystalline cellulose and silicified cellulose. In another preferred embodiment the one or more diluent(s) may also be selected from inorganic materials such as but not limited to calcium phosphate, calcium carbonate, calcium sulfate, calcium lactate, sodium chloride, magnesium oxide and magnesium carbonate.

In still another preferred embodiment, the pharmaceutical composition of the present invention is an oral solid dosage form such as a tablet or a capsule and most preferably the pharmaceutical composition is a tablet.

In another aspect, the present invention relates to the pharmaceutical composition as defined above for use as a medicament.

In still another aspect, the invention relates to the pharmaceutical composition as defined above for the treatment of a disease or disorder selected from dementia in Parkinson's disease, Huntington's chorea and Down's syndrome as well as symptoms associated with Alzheimer's disease.

In a preferred embodiment, the invention relates to the pharmaceutical composition as defined above for the treatment of symptoms associated with Alzheimer's disease.

In a further aspect, the invention relates to a method of treating symptoms associated with Alzheimer's disease comprising administering a predetermined and/or effective amount of crystalline form V of idalopirdine hydrochloride as defined herein and one or more acetylcholinesterase inhibitor(s) to a patient in need of such a treatment. In a preferred embodiment, the one or more acetylcholinesterase inhibitor(s) is/are selected from the group consisting of donepezil, rivastigmine, galantamine and tacrine.

### EXAMPLES

The following analytical methods and parameters have been applied for the generation of analytical data disclosed in the present invention:

### Powder X-ray diffraction

Powder X-ray diffraction was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-Theta with 40s per step (255 channels) in the angular range of 2° to 40° 2-Theta at ambient conditions. A typical precision of the 2-Theta values is in the range of ± 0.1 2-Theta. Thus, the reflection of crystalline form V of idalopirdine hydrochloride of the present invention that appears for example at 2.32° 2-Theta can appear between 2.22° and 2.42° 2-Theta on most X-ray diffractometers under standard conditions.

A representative powder X-ray diffractogram of crystalline idalopirdine hydrochloride form V of the present invention is displayed in Figure 1 and the corresponding reflection list from 2 to 30° 2-Theta is provided in Table 1 below.

**Table 1: Reflection list and corresponding relative intensities of crystalline form V of idalopirdine hydrochloride between 2.0 and 30.0° 2-Theta**

| Angle | Relative Intensity | Angle | Relative Intensity |
|---|---|---|---|
| [± 0.1 °2-Theta] | [%] | [±0.1 °2-Theta] | [%] |
| 2.30 | 100 | 21.62 | 17 |
| 4.63 | 33 | 23.07 | 18 |
| 6.94 | 3 | 24.29 | 6 |
| 13.95 | 16 | 24.62 | 6 |
| 17.20 | 37 | 25.24 | 83 |
| 17.71 | 17 | 25.97 | 12 |
| 17.95 | 23 | 26.91 | 4 |
| 18.78 | 33 | 27.33 | 7 |
| 19.69 | 24 | 28.04 | 11 |
| 20.35 | 4 | 29.32 | 5 |
| 21.00 | 12 | | |

### Fourier transform infrared spectroscopy

The FTIR spectrum was recorded (obtained) on an MKII Golden Gate™ Single Reflection Diamond ATR cell with a Bruker Tensor 27 FTIR spectrometer with 4 cm⁻¹ resolution at RT. To record a spectrum a spatula tip of a sample was applied to the surface of the diamond in powder form. Then the sample was pressed onto the diamond with a sapphire anvil and the spectrum was recorded. A spectrum of the clean diamond was used as background spectrum. A typical precision of the wavenumber values is in the range of about ± 2 cm⁻¹. Thus, the infrared peak of form V of idalopirdine hydrochloride that appears at 3426 cm⁻¹ can appear between 3424 and 3428 cm⁻¹ on most infrared spectrometers under standard conditions.

A representative FTIR spectrum of crystalline idalopirdine hydrochloride form V of the present invention is displayed in Figure 2 and the corresponding peak list from 4000 to 600 cm⁻¹ is provided in Table 2 below.

**Table 2: FTIR peak list of crystalline form V of idalopirdine hydrochloride between 4000 and 600 cm⁻¹**

| **Wavenumber** | **Wavenumber** |
|---|---|
| **[± 2 cm⁻¹]** | **[± 2 cm⁻¹]** |
| 3426 | 1135 |
| 2952 | 1120 |
| 2756 | 1091 |
| 1630 | 1045 |
| 1618 | 1019 |
| 1586 | 999 |
| 1502 | 951 |
| 1493 | 924 |
| 1452 | 879 |
| 1325 | 854 |
| 1299 | 839 |
| 1274 | 790 |
| 1258 | 764 |
| 1245 | 733 |
| 1220 | 713 |
| 1199 | 697 |
| 1182 | 683 |
| 1168 | 612 |

### Differential scanning calorimetry

Differential scanning calorimetry (DSC) was performed on a Mettler Polymer DSC R instrument. The sample (4.36 mg) was heated in a 40 microL aluminium pan with pierced aluminium lid from room temperature to 250 °C at a rate of 10 K/min. Nitrogen (purge rate 50 mL/min) was used as purge gas.

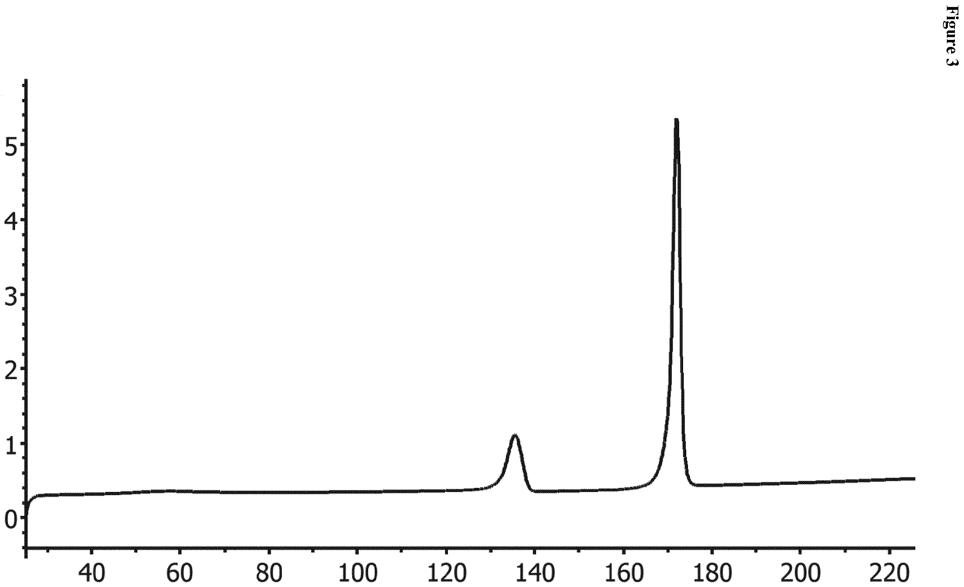

The DSC curve of form V is displayed in Figure 3 herein. It shows a first endothermic peak with an onset temperature of about 132 °C and a peak temperature of about 135 °C, which is due to the transformation of form V to form II. Finally, the second endothermic peak, which is visible in the DSC trace and shows an onset temperature of about 170 °C and a peak temperature of about 171 °C is caused by the melting of form II. Hence, according to DSC form V of the present invention is thermally stable up to a temperature of at least 120 °C.

### Thermogravimetric analysis

TGA was performed on a Mettler TGA/DSC 1 instrument. The sample (7.39 mg) was heated in a 100 microL aluminium pan closed with an aluminium lid, whereat the lid was automatically pierced at the beginning of the measurement. The sample was heated from room temperature to 250 °C at a rate of 10 K/min. Nitrogen (purge rate 50 mL/min) was used as purge gas.

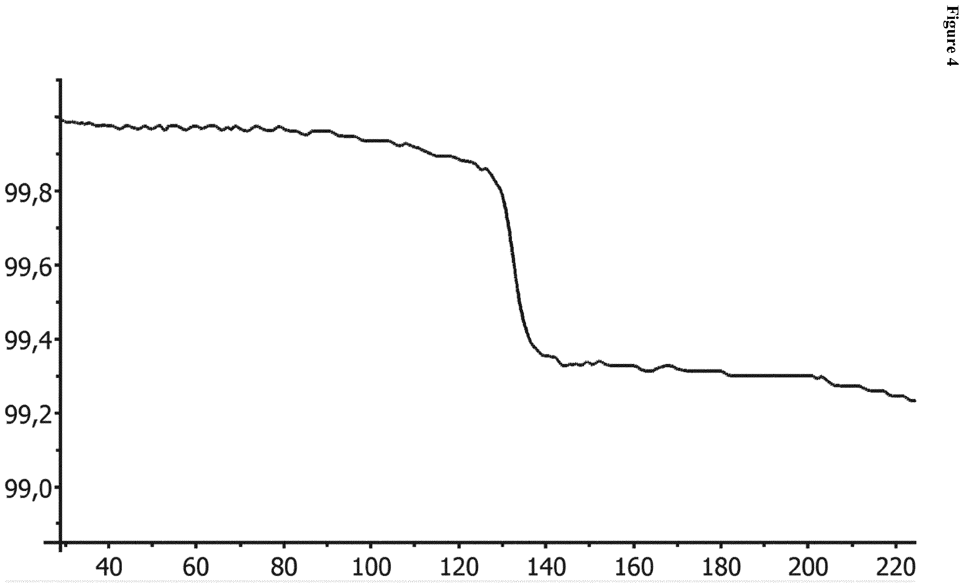

The TGA curve, which is displayed in Figure 4 herein, displays a mass loss of only about 0.6 w-% up to a temperature of 160 °C, confirming the presence of a non-solvated and anhydrous form.

### Gravimetric moisture sorption

Moisture sorption and desorption curves were recorded with an SPSx-1µ moisture sorption analyzer (ProUmid, Ulm). The measurement cycle was started at ambient RH of 45%. RH was then decreased to 5% in 5% steps, followed by a further decrease to 3% and to 0%. Afterwards RH was increased from 0% to 95% in a sorption cycle and subsequently decreased to 0 % in a desorption cycle each in 5% steps. Finally, RH was increased to 40% in 5% steps.

The time per step was set to a minimum of 2 hours and a maximum of 6 hours. If an equilibrium condition with a constant mass of ± 0.01 % within 1 hour was reached before the maximum time for all examined samples the sequential humidity step was applied before the maximum time of 6 hours. If no equilibrium was achieved the consecutive humidity step was applied after the maximum time of 6 hours. The temperature was 25 ± 0.1 °C.

The obtained moisture sorption and desorption curves are displayed in Figure 5 herein. A mass increase of only 0.5 w-% in the sorption cycle from 0 to 95% proves that form V is non-hygroscopic. In addition, the lack of a significant hysteresis between the sorption and desorption curve indicates that no structural changes occurred during the experiment, which was confirmed by powder X-ray diffraction, since the PXRDs before and after the GMS experiment were identical.

The following non-limiting examples are provided to further illustrative the present disclosure. They are not to be interpreted as being in any way limiting for the scope of the present invention.

### Example 1: Preparation of crystalline form V of idalopirdine hydrochloride

Idalopirdine hydrochloride (2.0 g, for example prepared according to the procedure disclosed in Example 4 of WO 2011/076212 A2) was dissolved in a mixture of 1,4-dioxane (27 mL) and n-heptane (25 mL) upon heating to reflux. The hot solution was transferred to a precooled reactor (jacket temperature -5 °C). Once the temperature of the solution reached 25 °C idalopirdine hydrochloride form V seed crystals (10 mg, prepared according to the procedure disclosed in Example 2 herein) were added. Subsequently, the temperature of the mixture was further decreased to 5 °C and stirred at the same temperature for 2 hours. Finally, the obtained crystals were collected by filtration, washed with cold n-heptane (10 mL) and dried under vacuum (30 mbar) at RT for 17 hours.

### Example 2: Preparation of idalopirdine hydrochloride form V seed crystals

A mixture of idalopirdine hydrochloride (0.2 g, for example prepared according to the procedure disclosed in Example 4 of WO 2011/076212 A2), 1,4-dioxane (2.5 mL) and n-heptane (2.5 mL) was heated to reflux. Additional 1,4-dioxane (0.2 mL) was added to the hot mixture, whereupon a clear solution was obtained. The hot solution was filtered through a syringe filter into a vial, which was precooled in an ice bath having a temperature of about 0 °C. The mixture was kept at a temperature of about 0 °C for 20 hours. Finally, the obtained crystals were collected by filtration

### Example 3: Accelerated stress test at 40 °C/75% RH

Idalopirdine hydrochloride form V was subjected to accelerated stress conditions of 40 °C and 75% RH in an open dish for one month. No change in the PXRD was observed, confirming the physical stability of idalopirdine hydrochloride form V. Form V also showed good chemical stability since it only showed an increase in total impurities of about 0.7 area% as determined by HPLC using the following method:

| | |
|---|---|
| HPLC system | : e.g. Agilent 1100 |
| Column | : YMC-Pack Pro C18 RS, 3 microm, 4.6 × 150 mm |
| Eluent system | : Eluent A: 3.88g sulfamic acid, fill up with water to 1000 g |
| | : Eluent B: 3.88 g sulfamic acid, fill up with water to 300 g and add 587 g acetonitrile |
| Injection volume | : 5 microL |
| Flow rate | : 0.8 mL/min |
| Oven temperature | : 40 °C |
| Stop time | : 16 min (post-time 3 min) |
| Detection | : lambda = 225 nm |
| System | : gradient |
| Gradient: | |

| **t (min)** | %A | %B |
|---|---|---|
| **0** | 90 | 10 |
| **10** | 0 | 100 |
| **15** | 0 | 100 |
| **16** | 90 | 10 |

The results of the stress test are again summarized in Table 3 below:

**Table 3: Chemical and physical stability of form V upon accelerated stress conditions**

| | **1 month at 40 °C/ 75% RH** | |
|---|---|---|
| **Idalopirdine hydrochloride form V of the present invention** | Increase of total impurities (HPLC at 225nm) | PXRD before and after |
| | +0.7 area% | unchanged |

### Comparative example 1: time-dependent solubility in 0.1 N aqueous HCl solution

The time dependent solubilities of idalopirdine hydrochloride form III and form V in 0.1 N HCl have been determined. Thereby, saturated solutions of both forms have been stirred at controlled temperature of (25 ± 1) °C and known aliquots have been taken after 5, 30 and 60 minutes. After filtering the solids, the idalopiridine concentrations of the remaining mother liquors were determined by HPLC using the same method as provided in Example 3 herein. The absolute solubility values were calculated from a calibration curve generated with solutions of known idalopirdine concentrations. According to powder X-ray diffraction the isolated solids did not undergo any solid state changes during the experiment.

**Table 4: Time dependent solubility of idalopirdine hydrochloride form III and form V in 0.1 N HCl at (25 ± 1) °C**

| | **Concentration** | |
|---|---|---|
| **Time** | **Form III** | **Form V** |
| **5 min** | 0.5 mg/mL | 0.7 mg/mL (+40%) |
| **30 min** | 0.6 mg/mL | 0.9 mg/mL (+50%) |
| **60 min** | 0.8 mg/mL | 0.9 mg/mL (+12.5%) |

As can be seen from the data presented in Table 4 above and from the dissolution curves provided in Figure 6, form V shows a significantly improved solubility in 0.1 N HCl compared to form III.

## Claims

1. A crystalline form of *N*-[2-(6-fluoro-1*H*-indol-3-yl)ethyl]-3-(2,2,3,3-tetrafluoropropoxy)benzylamine hydrochloride (form V) **characterized by** having:
a powder X-ray diffractogram comprising reflections at 2-Theta angles of (2.30 ± 0.1)° and (19.69 ± 0.1)°, when measured at a temperature in the range of from 20 to 30 °C with Cu-Kalpha_{1,2} radiation having a wavelength of 0.15419 nm.

2. The crystalline form according to claim 1, further **characterized by** having:
a Fourier transform infrared spectrum comprising peaks at wavenumbers of (3426 ± 2) cm⁻¹ and (1045 ± 2) cm⁻¹, when measured at a temperature in the range of from 20 to 30 °C with a diamond attenuated total reflection cell.

3. The crystalline form according to any one of claims 1 or 2, **characterized by** having a powder X-ray diffractogram not comprising a reflection at a 2-Theta angle of (19.97 ± 0.1)°, when measured at a temperature in the range of from 20 to 30 °C with Cu-Kalpha_{1,2} radiation having a wavelength of 0.15419 nm.

4. A composition comprising at least 90 w-% of the crystalline form of idalopirdine hydrochloride according to any one of claims 1 to 3, based on the weight of the composition.

5. Use of the crystalline form of idalopirdine hydrochloride according to any one of claims 1 to 3, or the composition according to claim 4, for the preparation of a pharmaceutical composition.

6. A pharmaceutical composition comprising the crystalline form of idalopirdine hydrochloride according to any one of claims 1 to 3, or the composition according to claim 4, and one or more pharmaceutically acceptable excipient(s).

7. The pharmaceutical composition of claim 6, wherein the one or more pharmaceutically acceptable excipient(s) comprises at least one diluent.

8. The pharmaceutical composition according to claim 7, which is a tablet or a capsule.

9. The pharmaceutical composition according to any one of claims 6 to 8 for use in the treatment of symptoms associated with Alzheimer's disease.

10. A process for the preparation of the crystalline form of idalopirdine hydrochloride of any one of claims 1 to 3 comprising the steps of:
(a) providing a suspension comprising idalopirdine hydrochloride, 1,4-dioxane and one or more antisolvent(s), wherein 1,4-dioxane is present in an amount of from 51 to 65 v-%, based on the total volume of the solvent/antisolvent mixture;
(b) heating the mixture provided in step (a) to a temperature of at least 60 °C in order to obtain a solution;
(c) cooling the solution provided in step (b) to a temperature of from 5 to 30 °C, preferably from 0 to 10 °C, in order to promote crystallization;

11. The process of claim 10, wherein the one or more antisolvent(s) is *n*-heptane.

12. The process according to any one of claims 10 or 11, comprising the additional step
(d) of seeding the mixture provided in step (c) with the crystalline form of idalopirdine hydrochloride according to any one of claims 1 to 3.

13. The process according to any one of claims 10 to 12 comprising the additional step
(e) of separating at least a part of the crystals obtained in step (c) or (d) from their mother liquor.

14. The process according to any one of claims 10 to 13 comprising the additional step (f) of drying the crystals obtained in any one of steps (c) to (e).
